Europäisches Patentamt

European Patent Office

Office européen des brevets

(11) Veröffentlichungsnummer: **0 045 362**
**B1**

## EUROPÄISCHE PATENTSCHRIFT

(45) Veröffentlichungstag der Patentschrift:
04.12.85

(21) Anmeldenummer: 81104311.6

(22) Anmeldetag: 04.06.81

(51) Int. Cl.⁴: **G 01 N 33/20,** G 01 N 21/67

(54) **Gerät zur spektralanalytischen Untersuchung der chemischen Zusammensetzung metallischer Werkstücke.**

(30) Priorität: 31.07.80 DE 3029038

(43) Veröffentlichungstag der Anmeldung:
10.02.82 Patentblatt 82/6

(45) Bekanntmachung des Hinweises auf die Patenterteilung:
04.12.85 Patentblatt 85/49

(84) Benannte Vertragsstaaten:
AT BE CH DE FR GB IT LI LU NL SE

(56) Entgegenhaltungen:
DE - A - 1 498 828
DE - A - 2 429 388
DE - A - 2 641 618
FR - A - 2 222 647
FR - A - 2 236 169

Die Akte enthält technische Angaben, die nach dem Eingang der Anmeldung eingereicht wurden und die nicht in dieser Patentschrift enthalten sind.

(73) Patentinhaber: KRUPP STAHL AKTIENGESELLSCHAFT, Postfach 1013 70, D-4630 Bochum 1 (DE)

(72) Erfinder: Kremer, Karl-Josef, Dr.Ing. Dipl.-Ing., Gutenbergstrasse 97b, D-5900 Siegen 21 (DE)
Erfinder: Brauner, Jochen Dr.rer.nat. Dipl.-Ing., Schöntalstrasse 2, D-5900 Siegen 21 (DE)
Erfinder: Glaubitz, Klaus-Dieter, Ing.-Grad., Ginsterweg 9, D-5912 Hilchenbach (DE)
Erfinder: Eckstein, Helmut, Gutehoffnungsring 40, D-5963 Wenden 5 (DE)
Erfinder: Brüggemann, Josef, Schwalbenweg 15, D-5963 Wenden 1 (DE)

(74) Vertreter: Patentanwaltsbüro Cohausz & Florack, Schumannstrasse 97, D-4000 Düsseldorf 1 (DE)

**Beschreibung**

Die Erfindung betrifft ein Gerät zur spektralanalytischen Untersuchung der chemischen Zusammensetzung metallischer Werkstücke mit einer auf das Werkstück aufsetzbaren Sonde, die eine insbesondere als Abfunkelektrode ausgebildete Verdampfungseinrichtung für das metallische Werkstück, mindestens einen gegenüber dem Werkstück wirksamen Abstandshalter für die Verdampfungseinrichtung und einen Empfangskopf für die Strahlung des durch die Verdampfungseinrichtung zum Leuchten gebrachten Metalldampfes aufweist, wobei der Abstandshalter eine einseitig offene, die Verdampfungseinrichtung aufnehmende Kammer ist, die mit ihrer Öffnung auf das Werkstück aufsetzbar ist.

Geräte zur spektralanalytischen Untersuchung der chemischen Zusammensetzung metallischer Werkstoffe dieser oder ähnlicher Art sind bekannt, z. B. aus DE-A1-2 429 388.

Bei einem aus DE-B-2 626 233 bekannten Gerät, das sich von dem Gerät der eingangs genannten Art nur dadurch unterscheidet, daß der Abstandshalter von zwei oder drei neben der als Abfunkelektrode ausgebildeten Verdampfungseinrichtung angeordneten Stiften besteht, wird die Strahlung des zum Leuchten gebrachten Metalldampfes von einem am Grundkörper der Sonde befestigten, flexiblen Lichtleitkabel, dessen freies Ende auf das Plasma des leuchtenden Metalldampfes ausgerichtet ist und den Empfangskopf bildet, zu einem Spektroskop übertragen, welches in einem mobilen Aggregat untergebracht ist. Dieses mobile Aggregat enthält auch die Energiequelle, in diesem Fall die Spannungsquelle für die Verdampfungseinrichtung.

Es hat sich nun gezeigt, daß äußere Einflüsse die Funktion und den Anwendungsbereich eines solchen Gerätes beeinflussen, nämlich Einflüsse, die die Stabilität eines Plasmas, wie die Stabilität eines Funkens bzw. eines Lichtbogens beeinträchtigen, ebenso wie Einflüsse, die auf die Übertragung der Strahlung von dem leuchtenden Metalldampf bis zum Empfangskopf einwirken. So wird die Stabilität des Funkens von unkontrollierbaren Luftströmungen beeinträchtigt, die sich auch durch die Anordnung der Abfunkelektrode in einer Kammer allein nicht ganz ausschließen lassen. Von besonderer Bedeutung ist, daß die Übertragung der Strahlung während des Durchganges durch Luft mehr oder weniger abgeschwächt wird. Die Abschwächung hängt dabei von der Wellenlänge des Lichtes ab und ist um so größer, je kürzer die Wellenlänge ist. Die bekannten, von Hand zu führenden Geräte eignen sich demnach nur für die Bestimmung von Komponenten mit größerer Wellenlänge, wie sie bei den metallischen Legierungsbestandteilen der zu untersuchenden Werkstoffe in der Regel vorliegen. Die analytische Bestimmung von Komponenten, die eine Strahlung kurzer Wellenlänge erzeugen, muß daher nach wie vor im Labor mit stationären Geräten vorgenommen werden. Dabei wird eine Probe des zu analysierenden Werkstückes in Schutzgasatmosphäre, meist in einer Argonatmosphäre, untersucht, wobei auch die im Wellenlängenbereich unter 250 nm liegenden Anteile bestimmt werden können.

Ein weiterer störender Nachteil der bekannten Geräte liegt darin, daß an dem Empfangskopf, einer Optik oder dem vorderen Ende eines Lichtleitkabels selbst sich Teile des verdampften Materials festsetzen, die die zu übertragende Strahlung schwächen, so daß von Zeit zu Zeit das Gerät gereinigt werden muß.

Eine Reihe der vorgenannten Nachteile treten bei einem bekannten Gerät der eingangs genannten Art nicht mehr auf, bei dem der Abstandshalter von einer einseitig offenen, die Verdampfungseinrichtung aufnehmenden Kammer gebildet ist, die mit ihrer Öffnung auf das Werkstück aufsetzbar ist (DE-A1-2 429 388). Bei diesem Gerät ist der Empfangskopf nicht außerhalb der Kammer, sondern innerhalb der Kammer hinter einer lichtdurchlässigen Scheibe angeordnet. Diese lichtdurchlässige Scheibe schützt zwar den Empfangskopf vor Verunreinigungen, doch muß die Scheibe, um funktionsfähig zu bleiben, wegen des sich darauf niederschlagenden Metalldampfes von Zeit zu Zeit gereinigt oder erneuert werden. Dieser Niederschlag ist trotz einer in der Kammerwandung vorgesehenen Öffnung zur Abführung von Gasen des Entladungsbogens beachtlich, weil durch die Kammer kein Spülgasstrom fließt.

Bei einem anderen bekannten Gerät (DE-A1-1 498 828), das ein typisches Laborspektrometer darstellt, ist eine Öffnung in einer ein Stativ bildenden, von der Außenluft abgeschlossenen und die Abfunkelektrode aufnehmenden Kammer von dem zu untersuchenden Werkstück abgedeckt. Außerhalb der Kammer hinter einem lichtdurchlässigen Fenster ist der Empfangskopf des Spektrometers angeordnet. Durch das die Kammer durchströmende Inertgas wird zwar der Teildampfniederschlag auf dem Fenster verringert, verhindert werden kann er jedoch nicht. Das bedeutet, daß wie bei dem vorigen Gerät das lichtdurchlässige Fenster von Zeit zu Zeit gereinigt oder erneuert werden muß.

Der Erfindung liegt die Aufgabe zugrunde, ein Gerät der eingangs genannten Art zu schaffen, das hinsichtlich der Stabilität des Funkens bzw. des Lichtbogens sowie hinsichtlich seines Einsatzbereiches und seiner Wartungsfreiheit verbessert ist. Ein solches Gerät soll vor allem im Freien die Schnellbestimmung eines Stoffes ermöglichen, z. B. des Kohlenstoffgehaltes eines Werkstückes, dessen Wellenlängenbereich deutlich unter 250 nm liegt.

Diese Aufgabe wird erfindungsgemäß dadurch gelöst, daß die Kammer in ihrer Wand ein offenes Fenster aufweist, vor dem außerhalb der Kammer der Empfangskopf angeordnet ist, und daß in die Kammer eine Leitung für Schutzgas mündet.

Beim erfindungsgemäßen Gerät wird in der Kammer eine definierbare Gasströmung erzeugt, die die Kammer ständig spült und mit ihrem aus der Kammer ausströmenden Gasstrom den Strahlengang zum Empfangskopf von die Messung störender Luft frei macht. Dadurch wird beim Arbeiten im Freien auch verhindert, daß Luftströmungen aus der Umgebung durch die Öffnung in das Innere der Kammer schlagen und dadurch die Stabilität des Funkens beeinträchtigen. Letzteres ist allerdings nur ein Nebeneffekt; viel bedeutender ist, daß die Erzeugung des Lichtbogens oder Funkens in einer Schutzgasatmosphäre, insbesondere in einer Argonatmosphäre, durchgeführt wird, so daß auch kurzwellige Strahlung, insbesondere die des Kohlenstoffes, zum Empfangskopf gelangen kann.

Eine besonders günstige Schutzgasführung ergibt sich dann, wenn die Leitung für das Schutzgas auf der dem Fenster gegenüberliegenden Seite der Verdampfungseinrichtung in die Kammer mündet. Günstig wirkt sich ferner aus, wenn die Mündung der Leitung für Schutzgas von der Öffnung der Kammer weiter entfernt ist als das Fenster, insbesondere dessen von der Öffnung am weitesten entfernte Rand.

Damit sich zwischen dem Empfangskopf und dem Fenster kein Luftfilm bildet, sol der Empfangskopf möglichst nahe am Fenster angeordnet sein. Dadurch wird erreicht, daß der Empfangskopf ständig vom Schutzgas angeströmt wird und sich in seinem unmittelbaren Bereich kein Luftfilm, der den Empfang der Strahlung beeinträchtigen könnte, bildet. Der Empfangskopf wird dabei auch ständig vom Schutzgas umströmt. Diese Strömung nimmt verdampftes Material mit, ohne daß es, wie bei bekannten Geräten, zu Aufdampfungen auf dem Empfangskopf kommt. Dadurch wird die Betriebszeit zwischen Reinigungsphasen verlängert.

Wenn es darum geht, in einer mit Schutzgasatmosphäre gefüllten, jedoch mit Öffnungen versehenen Kammer günstige Bedingungen zu schaffen, wird der Fachmann geneigt sein, in der Kammer durch kleine Querschnitte für die Öffnungen in der Kammer und möglichst große Mündungsquerschnitte für die Schutzgasleitung einen Überdruck zu schaffen. Es hat sich aber gezeigt, daß die gegenteiligen Maßnahmen von Vorteil sind. Sofern der Querschnitt der Leitung für das Schutzgas kleiner als der freie Querschnitt des Fensters ist, ergeben sich eine ruhige und gleichmäßige Spülung der Kammer sowie ein sanftes Anströmen des Empfangskopfes. Um auch im Oberflächenbereich des zu untersuchenden Werkstückes vorhandene Luftreste wegspülen zu können, empfiehlt es sich, daß der auf das Werkstück aufsetzbare Rand der Kammer nach außen führende Einkerbungen aufweist. Die Einkerbungen können von Zahnlücken einer Verzahnung gebildet werden. Sofern die Verdampfungseinrichtung als eine an eine Spannungsquelle anschließbare Abfunkelektrode und der Abstandshalter als Kontaktbrücke für die Übertragung des Stromes der Spannungsquelle ausgebildet sind, ergibt sich dabei der weitere Vorteil, daß die Zahnspitzen einen guten leitenden Kontakt zum Werkstück herstellen.

Ferner ist von Nutzen, wenn außerhalb der Kammer zu deren Kühlung Düsen für die Zufuhr eines Kühlmittels vorgesehen sind. Die Düsen umgeben die Kammer vorzugsweise ringförmig. Nach einer weiteren Ausgestaltung der Erfindung wird die Verdampfungseinrichtung, insbesondere die Abfunkelektrode, von einem mit Kühlmittel beaufschlagten Kühlkörper gehalten. Als Kühlmittelquelle kann ein im Gehäuse der Sonde vorgesehener Ventilator dienen.

Eine weitere Stabilisierung des Funkens ist dadurch möglich, daß in der Öffnung der Kammer eine zurückversetzte Blende aus Bornitrid angeordnet ist. Die Zurückversetzung dient dazu, eine kontrollierte Spülung durch das Schutzgas auf der Werkstückoberfläche zu gewährleisten.

Im folgenden wird die Erfindung anhand einer ein Ausführungsbeispiel darstellenden Zeichnung näher erläutert. Im einzelnen zeigt

Fig. 1 eine Sonde in Seitenansicht und teilweise im Axialschnitt,

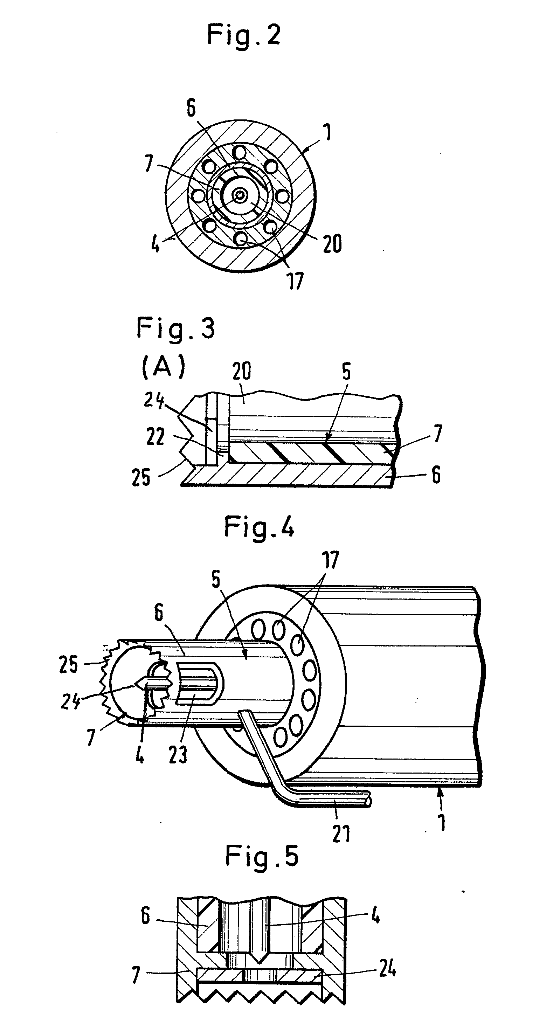

Fig. 2 die Sonde gemäß Fig. 1 im Querschnitt,

Fig. 3 einen Ausschnitt A der Sonde gemäß Fig. 1,

Fig. 4 dem vorderen Teil der Sonde gemäß Fig. 1 in perspektivischer Darstellung und

Fig. 5 den vorderen Teil der Sonde im Axialschnitt.

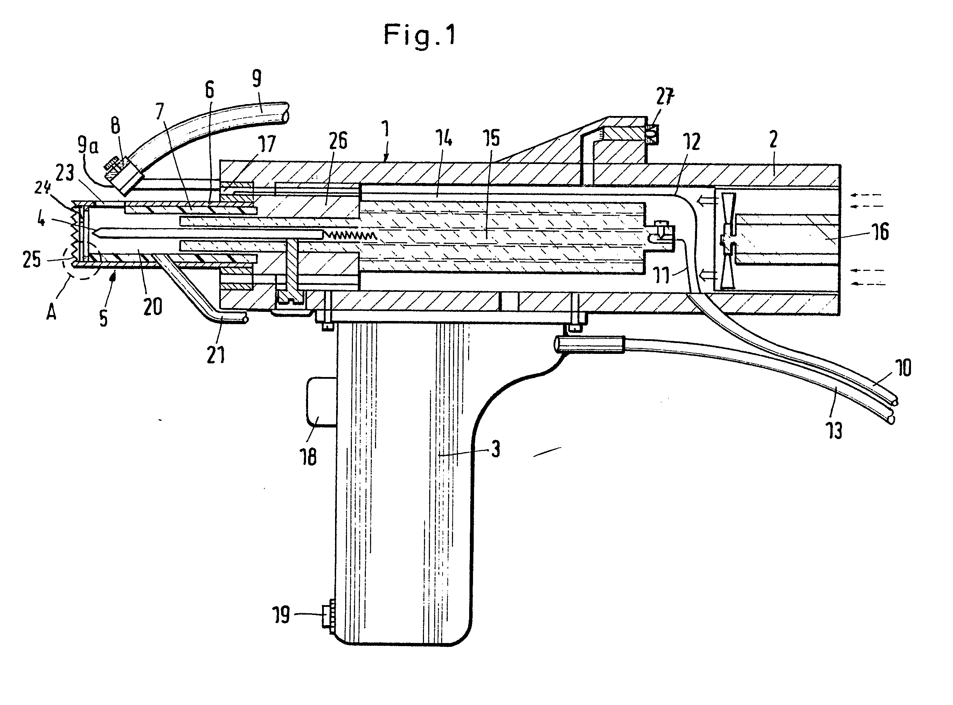

Die in Fig. 1 dargestellte Sonde eines Gerätes zur spektralanalytischen Untersuchung der chemischen Zusammensetzung metallischer Werkstücke hat als Grundkörper 1 die äußere Form einer Pistole und besteht aus einem Gehäuse 2 und einem Handgriff 3. Die Sonde ist über noch im einzelnen zu beschreibende flexible Leitungen mit einem nicht dargestellten Aggregat verbunden, in dem ein Spektralanalysegerät, eine Spannungsquelle, in der Regel elektronische Steuereinrichtungen und eine Schutzgasquelle, untergebracht sind.

Das Gehäuse 2 trägt am vorderen Ende eine Abfunkelektrode 4, die konzentrisch von einer hülsenförmigen, ebenfalls im Gehäuse 2 gehaltenen Kammer 5, umgeben ist. Die Kammer 5 besteht aus einem leitenden Außenmantel 6, deren vorderer Rand gezackt ist (Einkerbungen 25), und einem isolierenden Innenmantel 7. Die Abfunkelektrode 4 ist elektrisch mit einer Ader 11 eines zur Spannungsquelle des Aggregates führenden Kabels 10 verbunden, während der leitende Außenmantel 6 mit der Ader 12 des Kabels 10 verbunden ist. Hierbei wird die elektrische Verbindung zwischen der Ader 11 und der Abfunkelektrode 4 über einen koaxial im Gehäuse 2 angeordneten Kühlkörper 15 hergestellt, in dem die Abfunkelektrode 4 festgeklemmt ist. Die im Ringraum 14 zwischen dem Kühlkörper 15 und dem Gehäuse 2 geführte Ader 12 ist unmittelbar an eine Ringscheibe mit einem Kranz von Düsen 17 angeschlossen, die ihrerseits leitend den Außenmantel 6 umgibt. Ein im Gehäuse 2 sitzender und mit den Bohrungen 17 entsprechenden Bohrungen versehener Isolierkörper 26 hält den Kühlkörper 15 auf Abstand von der Wandung des Gehäuses 2.

Die Kammer 5 weist im rückwärtigen Bereich eine Mündung für eine Leitung 21 auf, über die von einer Leitung 13 gelieferte Schutzgas in das Innere 20 der Kammer 5 eingespeist werden kann. In der Nähe der Öffnung der Kammer 5 ist ein Fenster 23 vorgesehen, durch das das Schutzgas aus dem Innern 20 der Kammer 5 abströmen kann. Unmittelbar vor dem Fenster, außerhalb der Kammer 5, ist das als Empfangskopf 9a dienende und von einem Halter 8 getragene freie Ende eines Lichtleitkabels 9 von einem Halter gehalten, der seinerseits vom Gehäuse 2 getragen wird. Der Empfangskopf ist zum Fenster derart ausgerichtet, daß er die durch das Fenster fallende Strahlung aufnehmen kann und in der Strömung des aus dem Innern 20 der Kammer 5 austretenden Gases liegt. Die Leitung 21 für das Schutzgas und das Lichtleitkabel sind flexibel und mit dem Spektrometer bzw. der Schutzgasquelle des nicht dargestellten Aggregates verbunden.

Der vordere, auf das zu untersuchende Werkstück aufsetzbare Rand trägt Einkerbungen 25, über die bei auf dem Werkstück aufsitzender Sonde Schutzgas nach außen abströmen kann. Ferner sitzt im vorderen Bereich der Kammer 5, aber ein wenig zurückversetzt, eine Blende 24 aus Bornitrid, die sich auf einer Schulter 22 abstützt. In dem der Kammer 5 gegenüberliegenden Ende des Gehäuses ist ein Ventilator 16 angeordnet, der ein Kühlmittel gegen den Kühlkörper 15 und durch den Ringraum 14 zu den Düsen 17 bläst. Das aus den Düsen 17 austretende Kühlmittel beaufschlagt den Außenmantel 6 der Kammer 5.

Die Sonde ist über eine Steuerleitung 13 mit den entsprechenden Teilen des Aggregates verbunden. Die Steuerung und Kontrolle erfolgt mittels eines Startschalters 18 zum Einschalten der Energiezufuhr und einem Schalter 19, über den nach Beendigung des Meßvorganges das Aggregat für eine erneute Messung zurückgestellt werden kann.

Eine Kontrollanzeige 27 auf dem Gehäuse 2 zeigt der Bedienungsperson an, ob die durchgeführte Messung vorgegebenen Bedingungen entspricht.

Das erfindungsgemäße Gerät zeichnet sich durch seine Handlichkeit aus, denn die Bedienungsperson braucht nur die leichtgewichtige Sonde zu handhaben, die über flexible Leitungen mit dem schwergewichtigen Aggregat verbunden ist. Wegen der mit Schutzgas gefüllten Kammer und ihrer ständigen Durchspülung und der Umspülung des Empfangskopfes des Quarz-Lichtleitkabels 9 wird der Strahlengang von störenden Luftschichten freigehalten und verhindert, daß sich bereits nach kurzer Betriebszeit Dampf auf dem Empfangskopf ablagert, der die Messung stört. Die mit Schutzgas gefüllte Kammer ermöglicht auch die Arbeit im Freien, da sie den Funken vor Luftströmung schützt.

**Patentansprüche**

1. Gerät zur spektralanalytischen Untersuchung der chemischen Zusammensetzung metallischer Werkstücke mit einer auf das Werkstück aufsetzbaren Sonde, die eine insbesondere als Abfunkelektrode (4) ausgebildete Verdampfungseinrichtung für das metallische Werkstück, mindestens einen gegenüber dem Werkstück wirksamen Abstandhalter für die Verdampfungseinrichtung und einen Empfangskopf (9a) für die Strahlung des durch die Verdampfungseinrichtung zum Leuchten gebrachten Metalldampfes aufweist, wobei der Abstandhalter eine einseitig offene, die Verdampfungseinrichtung aufnehmende Kammer (5) ist, die mit ihrer Öffnung auf das Werkstück aufsetzbar ist, dadurch gekennzeichnet, daß die Kammer (5) in ihrer Wand (6, 7) ein offenes Fenster (23) aufweist, vor dem außerhalb der Kammer (5) der Empfangskopf (9a) angeordnet ist, und daß in die Kammer (5) eine Leitung (21) für Schutzgas mündet.

2. Gerät nach Anspruch 1, dadurch gekennzeichnet, daß die Leitung (21) für Schutzgas auf der dem Fenster (23) gegenüberliegenden Seite der Verdampfungseinrichtung (4) in die Kammer (5) mündet.

3. Gerät nach Anspruch 1 oder 2, dadurch gekennzeichnet, daß die Mündung der Leitung (21) für Schutzgas von der Öffnung der Kammer (5) weiter entfernt ist als das Fenster (23), insbesondere als dessen von der Öffnung am weitesten entfernter Rand.

4. Gerät nach einem der Ansprüche 1 bis 3, dadurch gekennzeichnet, daß der Abstand des Empfangskopfes (9a) vom Fenster (23) der Kammer (5) kleiner ist als die Höhe des Fensters (23).

5. Gerät nach einem der Ansprüche 1 bis 4, dadurch gekennzeichnet, daß der freie Querschnitt der Leitung (21) für Schutzgas kleiner als der freie Querschnitt des Fensters (23) ist.

6. Gerät nach einem der Ansprüche 1 bis 5, dadurch gekennzeichnet, daß der auf das Werkstück aufsetzbare Rand der Kammer (5) nach außen führende Einkerbungen (25) trägt.

7. Gerät nach einem der Ansprüche 1 bis 6, dadurch gekennzeichnet, daß bei einer Verdampfungseinrichtung, die als eine an einer Spannungsquelle angeschlossene Abfunkelektrode (4) ausgebildet ist, die Kammer (5) als Kontaktbrücke für die Übertragung des Stromes der Spannungsquelle ausgebildet ist.

8. Gerät nach einem der Ansprüche 1 bis 7, dadurch gekennzeichnet, daß außerhalb der Kammer (5) zu deren Kühlung Düsen (17) für die Zufuhr eines Kühlmittels vorgesehen sind.

9. Gerät nach Anspruch 8, dadurch gekennzeichnet, daß die Düsen (17) die Kammer (5) ringförmig umgeben.

10. Gerät nach einem der Ansprüche 1 bis 9, dadurch gekennzeichnet, daß die Verdampfungseinrichtung, insbesondere die Abfunkelektrode (4), von einem mit Kühlmittel beaufschlagten Kühlkörper (15) gehalten ist.

11. Gerät nach einem der Ansprüche 8 bis 10, dadurch gekennzeichnet, daß als Kühlmittelquelle im Gehäuse (2) der Sonde ein Ventilator (16) vorgesehen ist.

12. Gerät nach einem der Ansprüche 1 bis 11, dadurch gekennzeichnet, daß in der Öffnung der Kammer (5) eine zurückversetzte Blende (24) aus Bornitrid angeordnet ist.

## Claims

1. Device for the spectral analysis investigation of the chemical composition of metallic workpieces with a probe which is adapted to be placed on to the workpiece and which comprises a vaporising device, constructed especially as a sparking electrode, for the metallic workpiece, at least one spacing-maintaining element for the vaporising device effective relatively to the workpiece, and a reception head (9a) for the radiation of the metal vapour made to light up by the vaporising device, the spacing-maintaining element being a unilaterally open chamber (5) receiving the vaporising device, with the mouth of the said chamber adapted to be placed on to the workpiece, characterised in that the chamber (5) comprises in its wall (6, 7) an open window (23) in front of which the reception head (9a) is situated outside the chamber (5), and that a conduit (21) for protective gas opens into the chamber (5).

2. Device according to claim 1, characterised in that the conduit (21) for protective gas opens into the chamber (5) at that side of the vaporising device (4) which is opposite from the window (23).

3. Device according to claim 1 or 2, characterised in that the mouth of the conduit (21) for protective gas is situated further from the mouth of the chamber (5) than the window (23), especially further than that edge of said window which is furthest from the chamber mouth.

4. Device according to one of claims 1 to 3, characterised in that the spacing of the reception head (9a) from the window (23) of the chamber (5) is smaller than the height of the window (23).

5. Device according to one of claims 1 to 4, characterised in that the free cross-section of the conduit (21) for protective gas is smaller than the free cross-section of the window (23).

6. Device according to one of claims 1 to 5, characterised in that the edge of the chamber (5) which is applicable against the workpiece comprises outwardly directed indentations (25).

7. Device according to one of claims 1 to 6, characterised in that in the case of using a vaporising device which is constructed as a sparking electrode (4) connected to a voltage source the chamber (5) is constructed as a contact bridge for transmitting the current from the voltage source.

8. Device according to one of claims 1 to 7, characterised in that nozzles (17) for introducing a coolant are provided outside the chamber (5) for cooling the latter.

9. Device according to claim 8, characterised in that the nozzles (17) are arranged in a ring formation about the chamber (5).

10. Device according to one of claims 1 to 9, characterised in that the vaporising device, especially a sparking electrode (4), is held by a cooling body (15) on which coolant acts.

11. Device according to one of claims 8 to 10, characterised in that a fan (16) is provided as coolant source in the housing (2) of the probe.

12. Device according to one of claims 1 to 11, characterised in that a rearwardly offset diaphragm (24) of boron nitride is arranged in the mouth of the chamber (5).

## Revendications

1. Appareil pour l'analyse spectroscopique de la composition chimique de pièces de travail métalliques avec une sonde pouvant être posée sur la pièce de travail, qui présente un dispositif de vaporisation pour la pièce de travail métallique formée en particulier par une électrode (4), au moins un appui d'écartement agissant vis-à-vis de la pièce de travail pour le dispositif de vaporisation et une tête réceptrice (9a) pour le rayonnement de la vapeur métallique transformée en lumière par le dispositif de vaporisation, l'appui d'écartement étant une chambre (5) ouverte d'un côté et recevant le dispositif de vaporisation, qui peut être montée sur la pièce de travail par son ouverture, caractérisé par le fait que la chambre (5) présente dans sa paroi (6, 7) une fenêtre (23) ouverte devant laquelle est disposée la tête réceptrice (9a) à l'extérieur de la chambre (5), et qu'un conduit (21) pour du gaz de protection débouche dans la chambre (5).

2. Appareil selon la revendication 1, caractérisé par le fait que le conduit (21) pour du gaz de protection débouche dans la chambre (5) du côté du dispositif de vaporisation (4) opposé à la fenêtre (23).

3. Appareil selon la revendication 1 ou 2, caractérisé par le fait que la bouche du conduit (21) pour du gaz de protection est plus éloignée de l'ouverture de la chambre (5) que la fenêtre (23), en particulier que celle de l'ouverture au bord le plus éloigné.

4. Appareil selon l'une des revendications 1 à 3, caractérisé par le fait que la distance de la tête réceptrice (9a) à la fenêtre (23) de la chambre (5) est inférieure à la hauteur de la fenêtre (23).

5. Appareil selon l'une des revendications 1 à 4, caractérisé par le fait que la section transversale libre du conduit (21) pour du gaz de protection est inférieure à la section transversale libre de la fenêtre (23).

6. Appareil selon l'une des revendications 1 à 5, caractérisé par le fait que le bord de la chambre (5) pouvant être posé sur la pièce de travail porte des entailles (25) menant vers l'extérieur.

7. Appareil selon l'une des revendications 1 à 6, caractérisé par le fait que, pour un dispositif de vaporisation qui est formé par une électrode (4) reliée à une source de tension, la chambre (5) est formée en pont de contact pour la transmission du courant de la source de tension.

8. Appareil selon l'une des revendications 1 à 7, caractérisé par le fait que des ajutages (17) sont prévus en dehors de la chambre (5) pour son refroidissement pour l'amenée d'un agent réfrigérant.

9. Appareil selon la revendication 8, caractérisé par le fait que les ajutages (17) entourent annulairement la chambre (5).

10. Appareil selon l'une des revendications 1 à 9, caractérisé par le fait que le dispositif de vaporisation, en particulier l'électrode (4), est maintenu par un corps de refroidissement (15) alimenté en agent réfrigérant.

11. Appareil selon l'une des revendications 8 à 10, caractérisé par le fait qu'on prévoit, comme source d'agent réfrigérant, un ventilateur (16) dans le boîtier (2) de la sonde.

12. Appareil selon l'une des revendications 1 à 11, caractérisé par le fait qu'un écran inversé (24) en nitrure de bore est disposé dans l'ouverture de la chambre (5).

Fig.1

## Fig. 2

## Fig. 3

(A)

## Fig. 4

## Fig. 5